# EUROPEAN PATENT APPLICATION

(11) **EP 2 700 632 A1**
(43) Date of publication of application: **26.02.2014**
(21) Application number: 12773774.0
(22) Date of filing: 14.04.2012
(51) Int. Cl.: C07D 211/90, C07C 309/04, C07C 303/44, B01D 9/02, B01J 19/10

(54) **METHOD FOR PURIFICATION OF CALCIUM CHANNEL BLOCKERS OF DIHYDROPYRIDINE TYPE AND PREPARATION OF NANOPARTICLES THEREOF**

(30) Priority: 18.04.2011 CN 201110096853
(71) Applicant: Hefei Beini Medical Technology Company, Ltd, Hefei, Anhui 230051 (CN)
(72) Inventor: CHEN, Qiong, Hefei, Anhui 230051 (CN)
(74) Representative: Zwicker, Jörk
(86) International application number: PCT/CN2012/074045
(87) International publication number: WO 2012/142927

(57) **Abstract**

A method for purification calcium ion channel blocker medicament of dihydropyridine type and pharmaceutically acceptable salts thereof through the ultrasonic crystallization technology and direct preparation of nanoparticles thereof.

## Description

### Field of the invention

The present invention relates to the field of medicine, particularly, to a method for purification of dihydropyridine-type calcium channel blocker and pharmaceutically acceptable salts thereof through the ultrasonic crystallization technology and direct preparation of nanoparticles thereof.

### Background of the invention

After β-preceptor blocking agents, dihydropyridine-type calcium channel blocker is becoming another important kind of cardiovascular medicine which is developing rapidly in recent two decades, and is widely used for the treatment of hypertension, angina pectoris, arrhythmia such as supraventricular tachycardia, and hypertrophic cardiomyopathy in clinical practice, and some of which are also used for the prevention and treatment of cerebral ischemia and spasm as well as the reversion of early atherosclerosis.

Due to extremely low water solubility of the dihydropyridine-type calcium channel blockers, especially cilnidipine, lercanidipine, amlodipine and benidipine, the dibydropyridine-type calcium channel blockers or pharmaceutically acceptable salts thereof are usually smashed into nanoparticles, in order to allow their fast dissolution from the formulation. For example, a method for smashing benidipine hydrochloride into particles of 1.0-50.0 µm is provided by CN1794993A. In this patent mentioned above, large crystal particles are smashed into crystals with suitable size (from large to small size) by mechanical grinding. This method consumes large amount of energy and time, and results in a widely distribution of the crystal particle size.

Surprisingly, the present invention achieves purification of dihydropyridine-type calcium channel blockers (such as cilnidipine, lercanidipine, amlodipine, etc.) and pharmaceutically acceptable salts thereof by ultrasonic crystallization and nanoparticles of the crystal with suitable size are directly obtained. Distinguishing properties of the present invention are as follows: crystal obtained "from small to large size", narrow size distribution of the crystal due to crystallization by rapid equilibrium of the solvent in the solution, low energy and time consumption, labor saving, and easy preparation.

### Summary of the invention

In the present invention, dihydropyridine-type calcium channel blockers and pharmaceutically acceptable salts thereof are purified by ultrasonic crystallization technology and their nanoparticles can be prepared directly.

Generally, crystallization in solution is typically achieved by means of lowering temperature, allowing standing and the like, which is a very slow process consuming a long period of time. However, in the ultrasonic crystallization method provided by the present invention, a saturated or supersaturated solution is formed by altering temperature or the polarity of solvent, or by adding poor solvent, and subsequently nanoparticles with suitable size are obtained by ultrasound. Ultrasonic crystallization of the solution is carried out quickly and in an equilibrated fashion. The differences of crystallization solution, crystallization style, crystal growth rate, and mode of bonding between molecules inevitably lead to different molecular crystal forms and sizes.

To obtain the pharmaceutical nanoparticles described herein, dihydropyridine-type calcium channel blockers or pharmaceutically acceptable salts thereof, such as cilnidipine, lercanidipine and ammonia amlodipine, can be initially dissolved in a good solvent (such as methanol), and then saturated or supersaturated solution is formed by altering temperature or the polarity of solvent, or by adding poor solvent, subsequently, crystallization is promoted by ultrasonic intensification. After that, pharmaceutical nanoparticles with high purity can be directly obtained by routine operations including filtration (vacuum filtration), washing, drying and the like.

Good solvent and poor solvent for dissolving and/or forming pharmaceutical nanoparticles are typically selected from lower ketones, lower alcohols, lower ethers, lower esters, acetonitrile, dichloromethane, chloroform, acetic anhydride and common small molecule solvents. Preferably, the solvent is a single common solvent, such as methanol, ethanol, acetone, N, N-dimethyl formamide (DMF), acetonitrile, diethyl ether, dichloromethane, dimethyl sulfoxide (DMSO) and water, or the combination of two or three or more solvents above. The single solvent is preferably selected from methanol and acetonitrile. The combined solvent is mainly a combination of solvents dissolved in each other, preferably selected from, but not limited to, 9 combinations of two solvents, including methanol-acetone, methanol-water, methanol-ethanol, acetonitrile-water, acetonitrile-acetone, DMF-water, DMF-acetone, DMSO-water and DMSO-ethanol. For the combined solvent, the proportion of methanol to acetone is 0-100% : 100-0%; the proportion of methanol to water is 0-100%; the proportion of methanol to ethanol is 0-100%; the proportion of acetonitrile to water is 100-0% : 0-100%; the proportion of acetonitrile to acetone is 100-0% : 0-100%; the proportion of DMF to water is 0-100% : 100-0%; the proportion of DMF to acetone is 0-100% : 100-0%; the proportion of DMSO to water is 0-100% : 100-0%; the proportion of DMSO to ethanol is 0-100% : 100-0%.

The frequency for ultrasonic crystallization ranges from 20 kHz to 500 kHz, and preferably from 20 kHz to 100 kHz. The power of ultrasound ranges from 1 mW to 5000 W, and preferably from 1 W to 500 W. The intensity of ultrasound ranges from 0.1 mW/cm² to 500 W/cm², and preferably from 0.1 W/cm² to 50 W/cm². The duration of ultrasound ranges from 1 min to 24 hours, and preferably from 3 min to 120 min. The temperature for ultrasonic crystallization. ranges from -78°C to 100°C, and preferably from -5°C to 30°C. The size of the obtained nanoparticles of dihydropyridine-type calcium channel blocker ranges from 20 nm to 2000 nm, with the median size ranging from 200 nm to 1500 nm.

Specific embodiments are as follows:
Suitable amount of primary crystal of dihydropyridine-type calcium channel blocker or pharmaceutically acceptable salts thereof can be added into 0.1-40 folds of lower alcohol, and then is dissolved by heating under reflux; the temperature of the solution is lowered, and ultrasound treatment is performed to promote crystallization.

Alternatively, suitable amount of primary crystal of dihydropyridine-type calcium channel blocker or pharmaceutically acceptable salts thereof is added into 0.1-40 folds of methanol, and then dissolved by heating under reflux; 0.01-100 folds of ethanol is added, and the temperature of the solution is lowered, and ultrasound treatment is performed to promote crystallization.

Alternatively, suitable amount of primary crystal of dihydropyridine-type calcium channel blocker or pharmaceutically acceptable salts thereof is added into 0.1-40 folds of methanol, and then dissolved by heating under reflux; 0.01-100 folds of water is added, and the temperature of the solution is lowered, and ultrasound treatment is performed to promote crystallization.

Alternatively, suitable amount of primary crystal of dihydropyridine-type calcium channel blocker or pharmaceutically acceptable salts thereof is added into 0.1-40 folds of lower alcohol, and then dissolved by heating under reflux; 1/100 volume of water is added, and crystallization is carried out by virtue of ultrasound together with dripping water.

Alternatively, suitable amount of primary crystal of dihydropyridine-type calcium channel blocker or pharmaceutically acceptable salts thereof is added into 0.1-40 folds of lower alcohol, and then dissolved by heating under reflux; 0.1-100 fold of acetone is added, and the temperature of the solution is lowered, and ultrasound treatment is performed to promote crystallization.

Alternatively, suitable amount of primary crystal of dihydropyridine-type calcium channel blocker or pharmaceutically acceptable salts thereof is added into 0.1-40 folds of lower alcohol, and then dissolved by heating under reflux; 1/10 folds of acetone is added; when ultrasound is performed, acetone is dripped until crystallization begins, and subsequently ultrasound treatment continues for a further period of time (no more than 60 min).

Alternatively, suitable amount of primary crystal of dihydropyridine-type calcium channel blocker or pharmaceutically acceptable salts thereof is added into 0.1-15 folds of N,N-dimethyl formamide (DMF), and then dissolved by heating under reflux; 0.1-100 folds of water is added, and the temperature of the solution is lowered, and ultrasound treatment is performed to promote crystallization.

Alternatively, suitable amount of primary crystal of dihydropyridine-type calcium channel blocker or pharmaceutically acceptable salts thereof is added into 0.1-15 folds of DMF, and then dissolved by heating under reflux; 1/100 folds of water is added, and crystallization is carried out by virtue of ultrasound together with dripping water.

Alternatively, suitable amount of primary crystal of dihydropyridine-type calcium channel blocker or pharmaceutically acceptable salts thereof is added into 0.1-15 folds of DMF, and then dissolved by heating under reflux; 0.1-100 folds of ethanol is added, and the temperature of the solution is lowered, and ultrasound treatment is performed to promote crystallization.

Alternatively, suitable amount of primary crystal of dihydropyridine-type calcium channel blocker or pharmaceutically acceptable salts thereof is added into 0.1-15 folds of DMF, and then dissolved by heating under reflux; 1/10 folds of volume of ethanol is added; when ultrasound is performed, acetone is dripped until crystallization begins, and subsequently ultrasound treatment continues for a further period of time (no more than 60 min).

Alternatively, suitable amount of primary crystal of dihydropyridine-type calcium channel blocker or pharmaceutically acceptable salts thereof is added into 0.1-40 folds of acetonitrile, and then dissolved by heating under reflux; the temperature of the solution is lowered, and ultrasound treatment is performed to promote crystallization.

Alternatively, suitable amount of primary crystal of dihydropyridine-type calcium channel blocker or pharmaceutically acceptable salts thereof is added into 0.1-40 folds of acetonitrile, and then dissolved by heating under reflux; 0.01-100 folds of water is added, and the temperature of the solution is lowered, and ultrasound treatment is performed to promote crystallization.

Alternatively, suitable amount of primary crystal of dihydropyridine-type calcium channel blocker or pharmaceutically acceptable salts thereof is added into 0.1-40 folds of acetonitrile, and then dissolved by heating under reflux; 1/100 folds of water is added, and crystallization is carried out by virtue of ultrasound together with dripping water.

Alternatively, suitable amount of primary crystal of dihydropyridine-type calcium channel blocker or pharmaceutically acceptable salts thereof is added into 0.1-40 folds of acetonitrile, and then dissolved by heating under reflux; 0.1-10 fold of ethanol is added, and the temperature of the solution is lowered, and ultrasound treatment is performed to promote crystallization.

Alternatively, suitable amount of primary crystal of dihydropyridine-type calcium channel blocker or pharmaceutically acceptable salts thereof is added into 0.1-40 folds of acetonitrile, and then dissolved by heating under reflux; 0.1-10 fold of acetone is added, and the temperature of the solution is lowered, and ultrasound treatment is performed to promote crystallization.

Alternatively, suitable, amount of primary crystal of dihydropyridine-type calcium channel blocker or pharmaceutically acceptable salts thereof is added into 0.1-40 folds of acetonitrile, and then dissolved by heating under reflux; 0.1-10 fold ofacetone is added; when ultrasound is performed, acetone is dripped until crystallization begins, and subsequently ultrasound continues for a further period of time (no more than 60 min).

Alternatively, suitable amount of primary crystal of dihydropyridine-type calcium channel blocker or pharmaceutically acceptable salts thereof is added into 0.1-40 folds of acetonitrile, and then dissolved by heating under reflux; 0.1-10 folds of ethanol is added; when ultrasound is performed, acetone is dripped until crystallization begins, and subsequently ultrasound continues for a further period of time (no more than 5 min).

Alternatively, suitable amount of primary crystal of dihydropyridine-type calcium channel blocker or pharmaceutically acceptable salts thereof is added into 0.1-40 folds of acetonitrile, and then dissolved by heating under reflux; 0.1-100 folds of ethanol is added; then 0.1-1000 folds of water is slowly added, and ultrasound treatment is performed for 1-60 min.

Alternatively, suitable amount of primary crystal of dihydropyridine-type calcium channel blocker or pharmaceutically acceptable salts thereof is added into 0.1-20 folds of DMSO, and then dissolved by heating under reflux; 0.1-100 folds of ethanol is added, and the temperature of the solution is lowered, and ultrasound treatment is performed to promote crystallization.

Alternatively, suitable amount of primary crystal of dihydropyridine-type calcium channel blocker or pharmaceutically acceptable salts thereof is added into 0.1-20 folds of DMSO, and then dissolved by heating under reflux; 0.1-1000 folds of water is added, and ultrasound treatment is performed to promote crystallization.

### Detailed description of the invention

The present invention will be described in detail with reference to the following examples.

### Example 1

10 g primary crystal of amlodipine mesylate was added into 10 ml methanol, and then dissolved by heating under reflux. The solution was cooled down on an ice bath. After that crystallization was carried out by virtue of 150 W ultrasound for 10 min. The mixture was filtered under vacuum, and white crystal was collected with the crystal size shown in Fig. 1.

### Example 2

10 g primary crystal of cilnidipine was added into 10 ml methanol, and then dissolved by heating under reflux. The solution was cooled down on an ice bath. After that crystallization was carried out by virtue of 150 W ultrasound for 10 min. The mixture was filtered under vacuum, and faint yellow crystal was obtained.

### Example 3

10 g primary crystal of cilnidipine was added into 10 ml methanol, and then dissolved by heating under reflux. Subsequently, 12 ml water was added, and the solution was cooled down on an ice bath. Crystallization was carried out by virtue of 150 W ultrasound. After crystallization, ultrasound treatment was continued for further 2 min.

### Example 4

10 g primary crystal of cilnidipine was added into 10 ml methanol, and then dissolved by heating under reflux. Subsequently, 20 ml ethanol was added, and the solution was cooled down on an ice bath. Crystallization was carried out by virtue of 150 W ultrasound. After crystallization, ultrasound treatment was continued for further 5 min.

### Example 5

10 g primary crystal of lercanidipine hydrochloride was added into 10 ml methanol, and then dissolved by heating under reflux. Subsequently, 20 ml water was added, and the solution was cooled down on an ice bath. Crystallization was carried out by virtue of 150 W ultrasound. After crystallization, ultrasound treatment was continued for further 10 min to give faint yellow crystal.

### Example 6

10 g primary crystal of lercanidipine hydrochloride was added into 10 ml methanol, and then dissolved by heating under reflux. Subsequently, 3 ml water was added. 100 W ultrasound was then performed, and water was dripped into the solution during ultrasound treatment until crystallization was complete.

### Example 7

10 g primary crystal of amlodipine mesylate was added into 10 ml DMF, and then dissolved by heating under reflux. Subsequently, 3 ml water was added. 150 W ultrasound was then performed, and water was dripped into the solution until crystallization was complete.

### Example 8

10.1 g primary crystal of amlodipine mesylate was added into 11 ml DMF, and then dissolved by heating under reflux. Subsequently, 15 ml acetone was added, and the solution was cooled down on an ice bath. 200 W ultrasound was performed for crystallization.

### Example 9

10 g primary crystal of lercanidipine hydrochloride was added into 10 ml methanol, and then dissolved by heating under reflux. Subsequently, 10 ml acetone was added, and the solution was cooled down on an ice bath. 200 W ultrasound was performed for crystallization.

### Example 10

10 g primary crystal of lercanidipine hydrochloride was added into 10 ml methanol, and then dissolved by heating under reflux. Subsequently, 3 ml acetone was added, and 200 W ultrasound was performed. Acetone was dripped into the solution until crystallization began , and then ultrasound treatment was continued for further 3 min.

### Example 11

10 g primary crystal of cilnidipine was added into 10 ml methanol, and then dissolved by heating under reflux. Subsequently, 3 ml acetone was added, and 200 W ultrasound was performed. Acetone is dripped into the solution until crystallization begins , and then ultrasound treatment was continued for further 3 min.

### Example 12

10 g primary crystal of cilnidipine was added into 10 ml DMF, and then dissolved by heating under reflux. Subsequently, 15 ml water was added, and 200 W ultrasound was performed for crystallization.

### Example 13

10 g primary crystal of cilnidipine was added into 10 ml DMF, and then dissolved by heating under reflux. Subsequently, 5 ml water was added. 150 W ultrasound was then performed, and water was dripped into the solution until crystallization was complete.

### Example 14

10 g primary crystal of cilnidipine was added into 10 ml DMF, and then dissolved by heating under reflux. Subsequently, 20 ml ethanol was added, and the solution was cooled down on an ice bath. 200 W ultrasound was performed for crystallization.

### Example 15

10 g primary crystal of cilnidipine was added into 10 ml DMF, and then dissolved by heating under reflux. Subsequently, 3 ml acetone was added, and 200 W ultrasound was performed. Acetone was dripped into the solution until crystallization began , and then ultrasound treatment was continued for further 10 min.

### Example 16

10 g primary crystal of amlodipine mesylate was added into 10 ml acetonitrile, and then dissolved by heating under reflux. Subsequently, the solution was cooled down on an ice bath, and 150 W ultrasound was performed for crystallization.

### Example 17

10 g primary crystal of amlodipine mesylate medicament was added into 10 ml acetonitrile, and then dissolved by heating under reflux. Subsequently, 10 ml water was added. The solution was then cooled down on an ice bath, and 150 W ultrasound was performed for crystallization.

### Example 18

10 g primary crystal of amlodipine mesylate was added into 10 ml acetonitrile, and then dissolved by heating under reflux. Subsequently, 3 ml water was added. 150 W ultrasound was then performed, and water was dripped into the solution until crystallization was complete.

### Example 19

10 g primary crystal of cilnidipine was added into 10 ml acetonitrile, and then dissolved by heating under reflux. Subsequently, 10 ml ethanol was added, and the solution was cooled down on an ice bath. 200 W ultrasound was performed for crystallization.

### Example 20

10 g primary crystal of cilnidipine was added into 10 ml acetonitrile, and then dissolved by heating under reflux. Subsequently, 3 ml acetone was added, and 200 W ultrasound was performed. Acetone was dripped into the solution until crystallization began , and then ultrasound treatment was continued for further 15 min.

### Example 21

10.1 g primary crystal of cilnidipine was added into 10 ml acetonitrile, and then dissolved by heating under reflux. Subsequently, 3 ml acetone was added. 200 W ultrasound was then performed, and water was dripped into the solution until crystallization was complete.

### Example 22

10 g primary crystal of lercanidipine hydrochloride was added into 10 ml DMSO, and then dissolved by heating under reflux. Subsequently, 30 ml water was added. The solution was then cooled down on an ice bath, and 150 W ultrasound was performed for crystallization.

### Example 23

10 g primary crystal of lercanidipine hydrochloride was added into 10 ml DMSO, and then dissolved by heating under reflux. Subsequently, 20 ml ethanol was added. The solution was then cooled down on an ice bath, and 150 W ultrasound was performed for crystallization.

## Claims

1. A method for purification of dihydropyridine-type calcium channel blockers and pharmaceutically acceptable salts thereof and direct preparation of nanoparticles thereof by ultrasonic crystallization technology.

2. The ultrasonic crystallization method according to claim 1, comprising the following steps: saturated or supersaturated solution of dihydropyridine-type calcium channel blockers and pharmaceutically acceptable salts thereof is initially formed by altering temperature or the polarity of solvent, or by adding poor solvent, and then nanoparticles with suitable size are obtained by ultrasound.

3. Nanoparticles of dihydropyridine-type calcium channel blockers and pharmaceutically acceptable salts thereof obtained according to the method of claim 1 or claim 2, wherein the size of the nanoparticles ranges from 20 nm to 2000 nm, with the median size ranging from 200 nm to 1500 nm.

4. The ultrasonic crystallization method according to claim 1 or claim 2, wherein the temperature for ultrasonic crystallization ranges from -78°C to 100°C, and preferably from -5°C to 30°C.

5. The ultrasonic crystallization method according to claim 1 or claim 2, wherein the solvent for ultrasonic crystallization is selected from lower ketones, lower alcohols, lower ethers, lower acids, lower esters, dichloromethane, chloroform, acetic anhydride and common small molecule solvent, and preferably, the solvent is a single common solvent selected from acetone, ethanol, methanol, N, N-dimethyl formamide (DMF), acetonitrile, diethyl ether, dichloromethane, dimethyl sulfoxide (DMSO) and water, or a combination of two or three or more solvents mentioned above.

6. The ultrasonic crystallization method according to claim 1 or claim 2, wherein the ultrasound frequency for ultrasonic crystallization ranges from 20 kHz to 500 kHz, and preferably from 20 kHz to 100 kHz.

7. The ultrasonic crystallization method according to claim 1 or claim 2, wherein the ultrasound power for ultrasonic crystallization ranges from 1 mW to 5000 W, and preferably from 1 W to 500 W.

8. The ultrasonic crystallization method according to claim 1 or claim 2, wherein the ultrasound intensity for ultrasonic crystallization ranges from 0.1 mW/cm² to 500 V/cm², and preferably from 0.1 W/cm² to 50 W/cm².

9. The ultrasonic crystallization method according to claim 1 or claim 2, wherein the ultrasound duration for ultrasonic crystallization ranges from 1 min to 24 h, and preferably from 3 min to 120 min.

10. Nanoparticles of dihydropyridine-type calcium channel blockers and pharmaceutically acceptable salts thereof obtained according to the method of claim 1 or the method of claims 2-8.
